# EUROPEAN PATENT APPLICATION

(11) **EP 4 512 335 A1**
(43) Date of publication of application: **26.02.2025**
(21) Application number: 22966696.1
(22) Date of filing: 28.11.2022
(51) Int. Cl.: A61B 5/256, A61B 5/28, A61B 5/296, H01G 4/005

(54) **ELECTRODE STRUCTURE AND WEARABLE DEVICE**

(71) Applicant: Shenzhen Shokz Co., Ltd., Shenzhen, Guangdong 518108 (CN)
(72) Inventor: ZHOU, Xin, Shenzhen, Guangdong 518108 (CN); LIAO, Fengyun, Shenzhen, Guangdong 518108 (CN); QI, Xin, Shenzhen, Guangdong 518108 (CN)
(74) Representative: Wang, Bo
(86) International application number: PCT/CN2022/134676
(87) International publication number: WO 2024/113091

(57) **Abstract**

Embodiments of the present disclosure provide an electrode structure (100) including: a plurality of electrodes (110). Each of the plurality of electrodes (110) is flexibly connected to at least another of the plurality of electrodes (110) through a connector (120). The connection member (120) allows electrically conduction of the connected electrodes. Electrode structures (100) provided by embodiments of the present disclosure have a great elasticity coefficient range, and the electrode structures are adjustable.

## Description

### TECHNICAL FIELD

The present application relates to the field of signal collection, and in particular to an electrode structure and a wearable device.

### BACKGROUND

Smart wearable devices are growing tremendously as people focus on scientific exercise and physical health. The smart wearable devices monitor physiological signals of the human body (e.g., electrocardiogram (ECG) signals, electromyographic (EMG) signals, etc.) in real time to monitor the physiological condition of a user, and provide the user with exercise instructions, etc. Currently, the monitoring of the physiological signals such as the ECG signals and the EMG signals by the smart wearable devices mainly relies on electrodes. By integrating the electrodes on the clothing (tops, pants, straps, etc.) and fitting them to a user's skin, the physiological signals such as the ECG signals and the EMG signals are collected and analyzed. Due to the limitations of materials, it is difficult for the electrodes to have good elasticity (e.g., a poor elasticity), which leads to uncomfortable feelings such as a foreign body sensation, restricted movements, an impeded force generation, and inconvenient putting on or taking off when the user wears the wearable device.

Therefore, it is desired to provide an electrode structure with characteristics such as a great elastic range, adjustability, etc., which ensures that the human body has good wearing comfort when applied to the wearable device to collect physiological signals of the human body.

### SUMMARY

One embodiment of the present disclosure provides an electrode structure including: a plurality of electrodes, each of the plurality of electrodes is flexibly connected to at least another of the plurality of electrodes through a connector. The connector allows electrical conduction between the connected electrodes.

One of the embodiments of the present disclosure provides a wearable device including: a wearing portion, including a substrate fitting a user's body; at least two first electrodes spaced apart on the substrate and configured to fit a skin to collect physiological signals, respectively; and at least two second electrodes spaced apart on the substrate and electrically connected to each other through a connector, the at least two second electrodes being configured to fit the skin to provide a reference voltage for the physiological signals.

One of the embodiments of the present disclosure further provides a wearable device including: a wearing portion, including a substrate fitting a user's body; at least two electrode structures spaced apart on the substrate and configured to fit a skin to collect physiological signals. Each of the at least two electrode structures includes a plurality of electrodes, each of the plurality of electrodes is electrically connected to at least another of the plurality of electrodes through a connector, and the electrode structure is configured to collect electrical signals from a same target muscle of the user's body.

### BRIEF DESCRIPTION OF THE DRAWINGS

The present disclosure is further illustrated in terms of exemplary embodiments. These exemplary embodiments are described in detail with reference to the drawings. These embodiments are not limiting, and in these embodiments, the same numbering denotes the same structure, wherein
FIG. 1 is a schematic diagram illustrating an electrode structure according to some embodiments of the present disclosure;
FIG. 2 is a schematic diagram illustrating an electrode structure according to some embodiments of the present disclosure;
FIG. 3 is a schematic diagram illustrating an electrode structure according to some embodiments of the present disclosure;
FIG. 4 is a schematic diagram illustrating an electrode structure according to some embodiments of the present disclosure;
FIG. 5 is a schematic diagram illustrating an electrode structure according to some embodiments of the present disclosure;
FIG. 6 is a schematic diagram illustrating an electrode structure according to some embodiments of the present disclosure;
FIG. 7 is a schematic diagram illustrating a wearable device according to some embodiments of the present disclosure; and
FIG. 8 is a schematic diagram illustrating a wearable device according to some embodiments of the present disclosure.

### DETAILED DESCRIPTION

To more clearly illustrate the technical solutions of the embodiments of the present disclosure, the accompanying drawings required to be used in the description of the embodiments are briefly described below. Obviously, the accompanying drawings in the following description are only some examples or embodiments of the present disclosure, and it is possible for those skilled in the art to apply the present disclosure to other similar scenarios based on the accompanying drawings without creative labor. Unless obviously obtained from the context or the context illustrates otherwise, the same numeral in the drawings refers to the same structure or operation.

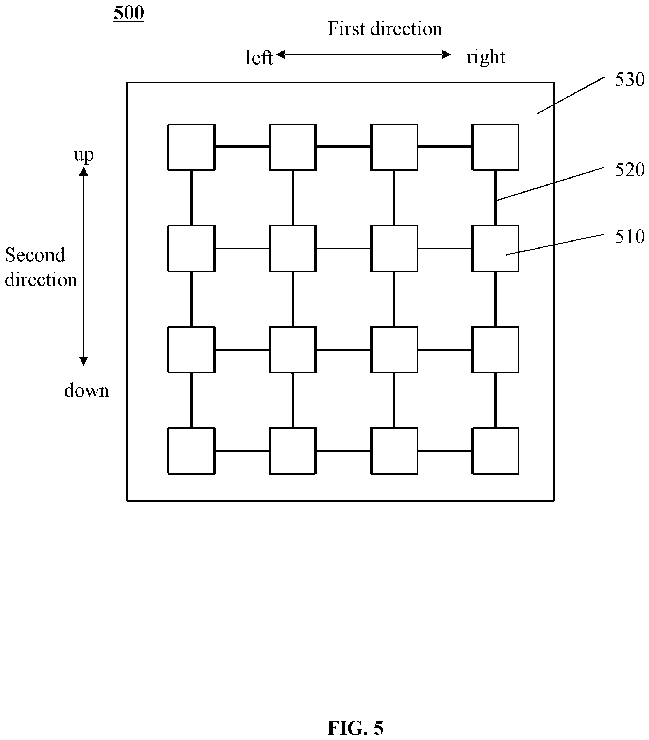

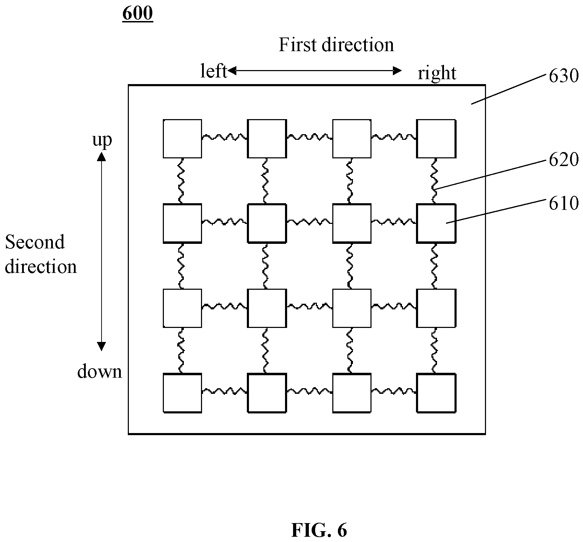

Embodiments of the present disclosure provide an electrode structure including a plurality of electrodes, each of the plurality of electrodes is flexibly connected to at least another of the plurality of electrodes through a connector. The connector allows electrical conduction between the connected electrodes. The plurality of electrodes in the electrode structure provided by the embodiments of the present disclosure are flexibly connected through the connector, so as to have a great deformable amount, so that when the electrode structure is applied to the wearable device to fit with a human skin to collect physiological signals (e.g., EMG signals, ECG signals, etc.) from the human body, the relatively great deformable amount of the electrode structure enables the user to put on and take off easily when wearing the wearable device, which does not limit movements (stretching, lifting legs, bending over, etc.) of the user, and doesn't hinder a user's strength, so as to ensure that the user has a good sense of comfort. In addition, the electrode structure provided in the embodiments of the present disclosure reduces a foreign body sensation caused by the electrode structure to a user when the user wears the electrode structure of the wearable device, and ensures that a position where the electrode structure is disposed on the wearable device is not easily damaged. In some embodiments, the deformable amount of the electrode structure provided by the embodiments of the present disclosure is a maximum deformation that the electrode structure can bear without irreversible damage (e.g., a fracture) under a force. The deformation of the electrode structure refers to the electrode structure undergoing deformation such as bending, expansion, etc. In some embodiments, by adjusting a count of connectors provided in a specific direction of the electrode structure in the embodiments of the present disclosure, the deformable amount of the electrode structure in the direction is adjusted such that the electrode structure can satisfy the usage requirements in more application scenarios. In some embodiments, the deformable amount of the electrode structure is increased by decreasing the elasticity coefficient of the electrode structure. It should be noted that in the present disclosure, the "flexible connection" refers to a connection that can achieve relative displacement (e.g., approaching or departing from each other) or rotation between two objects to be connected (e.g., two electrodes), and is capable of restoring to an initial shape of the connection under action of an external force or an elasticity of the connection. In some embodiments, the flexible connection is realized by an elastic member (e.g., a wire 520 shown in FIG. 5) connected between the two objects to be connected. The elastic member has a certain stretchable deformation capability, and the stretchable deformation of the elastic member causes a relative displacement between the two objects to be connected. In some embodiments, the flexible connection is realized by a structural member (e.g., a wire 620 shown in FIG. 6) connected between the two objects to be connected, having a specific length between the connected objects, and capable of unfolding, folding, or bending and meandering under the external force so as to cause a change in a redundancy length (i.e., a length that exceeds the specific length) of the structural member. The unfolding or folding of the structural member causes a relative displacement between the two objects to be connected. In some embodiments, the electrode structure provided by embodiments of the present disclosure is applied to a device that collects one or more physiological signals, for example, a smart wearable device (also referred to as a wearable device), a medical testing device, or a signal analysis device. In some embodiments, the smart wearable device is worn on various parts of a human body (e.g., a calf, a thigh, a waist, a back, a chest, shoulders, a neck, etc.) for collecting physiological signals from various parts of the user's body when the user is in different states, and the collected signals are further processed subsequently. In some embodiments, the smart wearable device includes a smart bracelet, smart footwear, smart glasses, a smart helmet, a smart watch, a smart dress, smart pants, a smart backpack, a smart accessory, etc., or any combination thereof. In some embodiments, the physiological signals are signals that are detectable and can reflect the state of the body, for example, include respiratory signals, ECG signals, EMG signals, blood pressure signals, temperature signals, and a variety of other signals.

The electrode structure provided by the embodiments of the present disclosure is described in detail below in connection with the accompanying drawings.

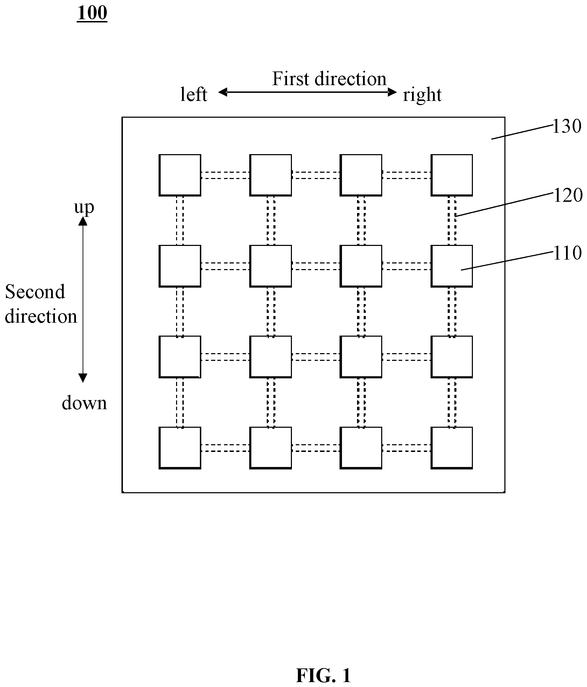

FIG. 1 is a schematic diagram illustrating an electrode structure according to some embodiments of the present disclosure.

As shown in FIG. 1, an electrode structure 100 includes a plurality of electrodes 110. Each of the plurality of electrodes 110 is flexibly connected to at least another of the plurality of electrodes 110 through a connector 120, and the connector 120 electrically conducts the connected electrodes. For example, each of the plurality of electrodes 110 has one or more adjacent electrodes 110, and each of the electrodes 110 is flexibly connected to the one or more of the electrodes 110 that are adjacent thereto through the connector 120. It is appreciated that the "adjacent electrodes" in the embodiments of the present disclosure refer to two or more electrodes that are adjacent to each other in a particular direction. For example, in a first direction shown in FIG. 1, each electrode has one or more adjacent electrodes, and in a second direction shown in FIG. 1, each electrode has one or more adjacent electrodes. In some embodiments, each electrode 110 is flexibly connected to all of the electrodes 110 adjacent thereto through a connector 120, as shown in FIG. 1. All of the electrodes 110 adjacent to a specific electrode 110 include all of the electrodes 110 adjacent to that electrode 110 along the first direction and all of the electrodes 110 adjacent to that electrode 110 along the second direction. In some embodiments, under the condition that all of the electrodes 110 are electrically conductive to each other, each of the electrodes 110 may be flexibly connected to only a portion of the electrodes 110 adjacent thereto through the connectors 120 to achieve the flexible connection. By realizing the flexible connection between each electrode 110 and the one or more electrodes 110 adjacent thereto through the connectors 120, on the one hand, it is equivalent to increasing a fitting area between the electrode structure 100 and human skin, thereby facilitating improvement of a signal-to-noise ratio (SNR), and on the other hand, a deformable amount of the electrode structure 100 is increased, and a better sense of comfort is ensured when the electrode structure 100 is used to fit the human skin to collect physiological signals of the human body. In some embodiments, the first direction is perpendicular to the second direction. For example, the electrode structure 100 is configured to fit the human skin to collect EMG signals. The first direction is a length direction of muscle fibers, and the second direction is perpendicular to the length direction of the muscle fibers. Merely by way of example, the electrode structure 100 is fitted to a position of leg muscles. The first direction is an axial direction of the leg, and the second direction is a circumferential direction of the leg. The electrode structure 100 may be fitted to a position of lumbar muscles, in which case the first direction is an axial direction of a lumbar spine, and the second direction is a circumferential direction of the lumbar.

In some embodiments, a plurality of electrodes 110 are distributed in a rectangular array as shown in FIG. 1, or an array such as a ring, a circle, etc. In some embodiments, the plurality of electrodes 110 are irregularly distributed, e.g., a relatively great count of electrodes 110 in the plurality of electrodes 110 are distributed centrally in positions that are more difficult to monitor or require focused attention, and a relatively small count of electrodes 110 in the plurality of electrodes 110 are dispersed in other positions. For ease of illustration, the present disclosure focuses on the electrode structure in which the plurality of electrodes are distributed in a rectangular array.

In some embodiments, as shown in FIG. 1, the electrode structure 100 further includes a substrate 130, and the plurality of electrodes 110 are arranged on the substrate 130 along the first direction and the second direction, respectively. The electrode structure 100 has different deformable amounts along the first direction and the second direction, respectively. In some embodiments, the electrode structure 100 has different elasticity coefficients along the first direction and the second direction, respectively, such that the electrode structure 100 has different deformable amounts along the first direction and the second direction, respectively. Specifically, the electrode structure 100 has a first elasticity coefficient along the first direction, and a second elasticity coefficient along the second direction. The first direction and the second direction are perpendicular to each other. In some embodiments, a count of electrodes 110 distributed in each row along the first direction in the electrode structure 100 is the same as a count of the electrodes 110 distributed in each column along the second direction in the electrode structure 100, as shown in FIG. 1. In some embodiments, the count of electrodes 110 distributed in each row along the first direction in the electrode structure 100 is different from the count of electrodes 110 distributed in each column along the second direction in the electrode structure 100. For example, the count of electrodes 110 distributed in each row along the first direction in the electrode structure 100 is greater or less than the count of electrodes 110 distributed in each column along the second direction in the electrode structure 100. In some embodiments, the substrate 130 is made of a flexible insulating material (e.g., resin, a soft PVC, silicone) and has a rectangular, circular, or other irregular shape. In some embodiments, the electrodes 110 are fixedly connected to the substrate 130 through pasting, snapping, welding, etc. In some embodiments, when the electrode structure 100 is applied to a wearable device, the substrate 130 is a portion of a wearing portion (e.g., top, pants, belt, strap, etc.) of the wearable device that is to be worn by the user, or the substrate 130 is disposed separately from the wearing part and then fixedly connected to a surface of the wearing portion that is fitted to the user's body through pasting, snapping, welding, etc., so that the electrode structures 100 are fixed on the wearing portion to fit the user's skin to collect the physiological signals. In some embodiments, the deformable amount of the substrate 130 (or the deformable amount of the wearing portion) is consistent or approximately consistent with the deformable amount of the connector 120, which reduces the discomfort caused by the electrode structure to the user when the user wears the wearable device, and ensures that a position where the electrode structure 100 is disposed on the wearing portion is not easily damaged. It should be noted that in some embodiments, the electrode structure 100 may not include the substrate 130, and the flexible connection between each electrode 110 and the one or more adjacent electrodes 110 thereof is realized only through the connectors 120.

In some embodiments, the electrodes 110 are sheet-like structures, and the electrodes 110 may have regular shapes such as circles, ovals, rectangles, diamonds, or other irregular shapes. In some embodiments, the electrodes 110 are made of single materials, such as metal fabric electrodes, conductive silicon electrodes, hydrogel electrodes, metal electrodes, etc. In some embodiments, the electrodes 110 are metallic fabric electrodes or conductive silicon electrodes. The metallic fabric electrodes have a much lower resistivity. When the electrode structure 100 is used to fit to the human skin to collect the physiological signals, the impedance of the metallic fabric electrodes, as well as the contact impedance with the skin, is also relatively low. The smaller the thickness of the metal fabric electrodes, the smaller the impedance and the contact impedance with the skin. In some embodiments, the smaller the contact impedance between the electrodes 110 and the skin, the lower the contact impedance between the electrode structure 100 and the skin, and the higher a strength of the physiological signals collected. In some embodiments, when using the metal fabric electrodes to collect the physiological signals, the thickness of the metal fabric electrode is in a range of 10 µm-5 mm. In some embodiments, the thickness of the metal fabric electrode is in a range of 100 µm-3 mm. In some embodiments, the thickness of the metal fabric electrode is in a range of 500 µm-2 mm. In some embodiments, the electrodes 110 are electrodes formed by a superposition of different materials, such as the electrodes formed by a metal fabric material and a conductive silicon material, which have a low contact impedance between the electrodes and the skin. A conductive silicon of the electrodes that is in contact with the skin has advantages such as being skin-friendly and washing-resistant, thereby avoiding a discomfort feeling to the human body brought about by the contact between the electrode and the skin.

In some embodiments, the first elasticity coefficient of the electrode structure 100 along the first direction and/or the second elasticity coefficient of the electrode structure 100 along the second direction is adjusted by controlling the size of the electrodes 110 to adjust the deformable amounts of the electrode structure 100 along the first direction and/or the second direction. In some embodiments, the first elasticity coefficient of the electrode structure 100 along the first direction and/or the second elasticity coefficient of the electrode structure 100 along the second direction are adjusted by controlling a ratio of a maximum size of the electrode 110 along the first direction to a maximum size of the electrode 110 along the second direction. In some embodiments, when the maximum size of the electrode 110 along the first direction is greater than the maximum size of the electrode 110 along the second direction, the first elasticity coefficient of the electrode structure 100 along the first direction is less than the second elasticity coefficient of the electrode structure 100 along the second direction, and the deformable amount of the electrode structure 100 along the first direction is greater than the deformable amount of the electrode structure 100 along the second direction. In some embodiments, the ratio of the maximum size of the electrode 110 along the first direction to the maximum size of the electrode 110 along the second direction is in a range of 0.1-10. In some embodiments, the ratio of the maximum size of the electrode 110 along the first direction to the maximum size along the second direction is in a range of 0.5-9. In some embodiments, the ratio of the maximum size of the electrode 110 along the first direction to the maximum size along the second direction is in a range of 1-8. In some embodiments, the ratio of the maximum size of the electrode 110 along the first direction to the maximum size of the electrode 110 along the second direction is designed according to an elasticity requirement of the electrode structure 100 in an application scenario. For example, if a smaller deformable amount of the electrode structure along the second direction causes an obvious effect on improving the comfort of the human body when the electrode structure 100 fits with the human skin, the ratio of the maximum size of the electrode 110 along the first direction to the maximum size of the electrode 110 along the second direction is designed to be greater.

In some embodiments, by controlling a count and/or an area of the electrodes 110 in the electrode structure 100, and/or a ratio of a total area of the electrode structure 100 to a sum of the areas of all the electrodes 110, the first elasticity coefficient of the electrode structure 100 along the first direction and/or the second elasticity coefficient of the electrode structure 100 along the second direction is adjusted to adjust the deformable amounts of the electrode structure 100 along the first direction and/or the second direction.

In some embodiments, the greater the count of the electrodes 110, the greater the first elasticity coefficient of the electrode structure 100 along the first direction and/or the second elasticity coefficient of the electrode structure 100 along the second direction, and the smaller the deformable amount of the electrode structure 100. For example, for a constant total area of the electrode structure 100, the greater the count of electrodes 110, the smaller the distance between adjacent electrodes 110, the smaller the length of the connector 120 connected between two adjacent electrodes 110 (e.g., the maximum size of the connector 120 along the first direction or the second direction), the greater the elasticity coefficient of the connector 120, the smaller the deformable amount of the connector 120, and the smaller the deformable amounts of the electrode structure 100 along the first direction and/or the second direction. To ensure that the electrode structure 100 has a relatively great deformation amount, in some embodiments, the count of the electrodes 110 in the electrode structure 100 is in a range of 2-1,000. In some embodiments, the count of the electrodes 110 in the electrode structure 100 is in a range of 10-1000. In some embodiments, the count of the electrodes 110 in the electrode structure 100 is in a range of 20-1000. In some embodiments, the count of the electrodes 110 in the electrode structure 100 is in a range of 100-1000. In some embodiments, the count of the electrodes 110 in the electrode structure 100 is in a range of 500-1000. It should be noted that the count of electrodes shown in FIG. 1 is intended as an example only, and is not intended to be limiting.

In some embodiments, the smaller the area of each electrode 110 in the electrode structure 100, the smaller the first elasticity coefficient of the electrode structure 100 along the first direction and/or the second elasticity coefficient of the electrode structure 100 along the second direction, and the greater the deformable amount of the electrode structure 100 along the first direction or the second direction. For example, for a constant total area of the electrode structure 100, the smaller the area of the electrodes 110, the greater the distance between adjacent electrodes 110, the greater the length of the connector 120 connected between the adjacent electrodes 110 (e.g., the maximum size of the connector 120 along the first direction or the maximum size of the connector 120 along the second direction), the smaller the elasticity coefficient of the connector 120, the greater the deformable amount of the connector 120, and the greater the deformable amounts of the electrode structure 100 along the first direction and/or the second direction. To ensure that the electrode structure 100 has a relatively great deformable amount, in some embodiments, the area of each electrode 110 in the electrode structure 100 is in a range of 1 mm²-20 mm². In some embodiments, the area of each electrode 110 in the electrode structure 100 is in a range of 2 mm²-18 mm². In some embodiments, the area of each electrode 110 in the electrode structure 100 is in a range of 2 mm²-15 mm². In some embodiments, the area of each electrode 110 in the electrode structure 100 is in a range of 2 mm²-10 mm².

In some embodiments, the greater the ratio of the area of the electrode structure 100 to the sum of the areas of all the electrodes 110, the smaller the first elasticity coefficient of the electrode structure 100 along the first direction and/or the second elasticity coefficient of the electrode structure 100 along the second direction, and the greater the deformable amounts of the electrode structure 100 along the first direction and/or along the second direction. For example, the greater the ratio of the area of the electrode structure 100 to the sum of the areas of all the electrodes 110, the greater the distance between adjacent electrodes 110, the greater the length of the connector 120 connected between the adjacent electrodes 110 (e.g., the maximum size of the connector 120 along the first direction or the maximum size of the connector 120 along the second direction), the smaller the elasticity coefficient of the connector 120, the greater the deformable amount of the connector 120, and the greater the deformable amounts of the electrode structure 100 along the first direction and/or the second direction. The area of the electrode structure 100 refers to an area of an overall shape presented by the electrode structure 100. To ensure that the electrode structure 100 has a relatively great deformable amount, in some embodiments, the ratio of the area of the electrode structure 100 to the sum of the areas of all the electrodes 110 is in a range of 1-1000. In some embodiments, the ratio of the area of the electrode structure 100 to the sum of the areas of all the electrodes 110 is 2-100. In some embodiments, the ratio of the area of the electrode structure 100 to the sum of the areas of all the electrodes 110 is in a range of 2-10. In some embodiments, the ratio of the area of the electrode structure 100 to the sum of the areas of all the electrodes 110 is in a range of 4-10.

In some embodiments, the deformable amounts of the electrode structure along the first direction and/or the second direction may be adjusted by controlling the count of connectors disposed in the electrode structure along the first direction and the count of connectors disposed in the electrode structure along the second direction. In some embodiments, the first elasticity coefficient of the electrode structure 100 along the first direction and/or the second elasticity coefficient of the electrode structure 100 along the second direction may be adjusted by controlling the count of the connectors disposed along the first direction of the electrode structure and the count of the connectors disposed along the second direction of the electrode structure, thereby adjusting the deformable amounts of the electrode structure along the first direction and/or the second direction.

In some embodiments, as shown in FIG. 1, the count of connectors 120 of the electrode structure 100 along the first direction is the same as the count of connectors 120 of the electrode structure 100 along the second direction, and the elasticity coefficient of the electrode structure 100 along the first direction and the second elasticity coefficient of the electrode structure 100 along the second direction are the same or approximately the same, so that the deformable amounts of the electrode structure 100 along the first direction and the second direction are the same or approximately the same. In some embodiments, the first elasticity coefficient (the deformable amount) of the electrode structure 100 along the first direction and the second elasticity coefficient (the deformable amount) of the electrode structure 100 along the second direction being approximately the same refers to that the difference between the first elasticity coefficient and the second elasticity coefficient is in a range of 1%-10%. Specifically, as shown in FIG. 1, adjacent electrodes 110 disposed along the first direction in each row are connected through the connectors 120, and adjacent electrodes 110 disposed along the second direction in each column are connected through the connectors 120. The count of the connectors 120 of the electrode structure 100 disposed along the first direction and the count of connectors 120 of the electrode structure along the second direction 100 are both 12. In some embodiments, under the condition that all the electrodes 110 are capable of being electrically conductive to each other, only a portion of adjacent electrodes 110 disposed along the first direction in the electrode structure 100 are connected through the connector 120, and a portion of adjacent electrodes 110 disposed along the second direction in the electrode structure 100 are connected through the connector 120, as long as the count of connectors 120 of the electrode structure 100 disposed along the first direction is the same as the count of connectors 120 of the electrode structure 100 disposed along the second direction. The electrodes 110 being electrically conductive refers to that any two electrodes 110 in the electrode structure 100 are connected to each other directly or indirectly through one or more connectors 120 to realize an electrical conduction. It should be noted that the count of electrodes 110 and the count of the connectors 120 shown in FIG. 1 is only an example and is not intended to be limiting, and when the count of the electrodes of the electrode structure is any other count, the count of the connectors of the electrode structure along the first direction and the count of the connectors 120 of the electrode structure along the second direction are designed with reference to how the count of the connectors 120 of the electrode structure 100 along the first direction and the count of the connectors 120 of the electrode structure 100 along the second direction are designed.

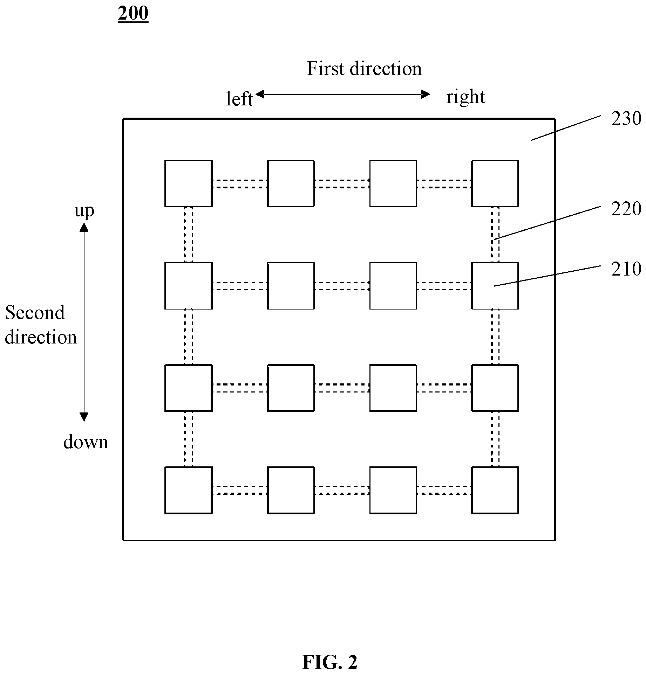

FIG. 2 is a schematic diagram illustrating an electrode structure according to some embodiments of the present disclosure.

In some embodiments, as shown in FIG. 2, a count of connectors 220 of an electrode structure 200 disposed along a first direction is greater than a count of the connectors 220 of the electrode structure 200 disposed along a second direction, and a first elasticity coefficient of the electrode structure 200 along the first direction is greater than a second elasticity coefficient of the electrode structure 200 along the second direction, so that a deformable amount of the electrode structure 200 along the first direction is smaller than a deformable amount of the electrode structure 200 along the second direction. Electrodes 210, the connectors 220, and a substrate 230 in the electrode structure 200 are similar to the electrodes 110, the connectors 120, and the substrate 130 in the electrode structure 100, respectively. More descriptions regarding the electrodes 210, the connectors 220, and the substrate 230 in the electrode structure 200 may be found in the relevant descriptions regarding the electrodes 110, the connectors 120, and the substrate 130 in the electrode structure 100, which are not repeated herein.

In some embodiments, adjacent electrodes 210 disposed along the first direction in each row in the electrode structure 200 are connected to each other through the connectors 220, and a portion of adjacent electrodes 210 disposed along the second direction are connected to each other through the connectors 220, such that the count of the connectors 220 of the electrode structure 200 disposed along the first direction is greater than the count of the connectors 220 of the electrode structure 200 disposed along the second direction, and the first elasticity coefficient of the electrode structure 200 along the first direction is greater than the second elasticity coefficient of the electrode structure 200 along the second direction. In such cases, the deformable amount of the electrode structure 200 along the first direction is smaller than the deformable amount of the electrode structure 200 along the second direction. Specifically, when a count of rows disposed along the first direction and connected through the connectors 220 in the electrode structure 200 is greater than a count of columns disposed along the second direction and connected through the connectors 220 in the electrode structure 200, the first elasticity coefficient of the electrode structure 200 along the first direction is greater than the second elasticity coefficient of the electrode structure 200 along the second direction, such that the deformable amount of the electrode structure 200 along the first direction is smaller than the deformable amount of the electrode structure 200 along the second direction. For example, as shown in FIG. 2, the count of the connectors 220 along the first direction in the electrode structure 200 is 12, and the count of the connectors 220 along the second direction in the electrode structure 200 is 6. The 12 connectors 220 disposed along the first direction in the electrode structure 200 are used to connect all two adjacent electrodes 210 disposed along the first direction in the electrode structure 200, and 3 connectors 220 of the 6 connectors 220 disposed along the second direction in the electrode structure 200 are used to connect 4 electrodes 210 distributed in a first column from left to right in the electrode structure 200, and the other 3 connectors 220 are used to connect 4 electrodes 210 distributed in a last column from left to right in the electrode structure 200. In some embodiments, the count of connectors 220 disposed along the second direction in the electrode structure 200 is 3. Under the condition that all of the electrodes 210 are capable of being electrically conductive to each other, the 3 connectors 220 of the electrode structure 200 are used to connect 4 electrodes distributed in the same column, or used to connect two adjacent electrodes 210 distributed in different columns in the electrode structure 200, wherein 4 electrodes 210 in electrodes 210 connected by the 3 connectors 220 are distributed in different rows. For example, under the condition that all the electrodes 210 are capable of being electrically conductive to each other, a first connector 220 of the 3 connectors 220 is used to connect two adjacent electrodes 210 distributed in a first column, and a second connector 220 of the 3 connectors 220 is used to connect two adjacent electrodes 210 distributed in a second column, and a third connector 220 of the 3 connectors 220 is used to connect two adjacent electrodes 210 distributed in a third column. 4 electrodes 210 of the electrodes 210 connected by the 3 connectors need to be distributed in different rows such that all the electrodes 210 are electrically conductive. In some embodiments, under the condition that all the electrodes 210 are capable of being electrically conductive to each other, the count of the connectors 220 disposed along the second direction in the electrode structure 200 is greater than 3 and less than the count of connectors 220 disposed along the first direction in the electrode structure 200. For example, the count of the connectors 220 disposed along the second direction in the electrode structure 200 is 4, 5, 6, 7, etc. In some embodiments, under the condition that all the electrodes 210 are capable of being electrically conductive to each other, only a portion of adjacent electrodes 210 disposed along the first direction in the electrode structure 200 are connected to each other through the connectors 120, as long as the count of the connectors 220 disposed along the first direction is greater than the count of the connectors 220 disposed along the second direction. It is understood that the electrodes 210 in each row of the electrode structure 200 refer to a plurality of electrodes 210 disposed along a direction parallel to the first direction and in a same straight line in the electrode structure 200. The electrodes 210 in each column refer to a plurality of electrodes 210 disposed along a direction parallel to the second direction and in the same straight line in the electrode structure 200. It is noted that as shown in FIG. 2, the electrodes 210 and the count of connectors 220 are only for examples and are not intended to be limiting, and when the count of the electrodes in the electrode structure is another count, the count of the connectors disposed along the first direction and the count of the connectors disposed along the second direction in the electrode structure are designed with reference to the count of the connectors 220 disposed along the first direction and the count of the connectors 220 disposed along the second direction in the electrode structure 200.

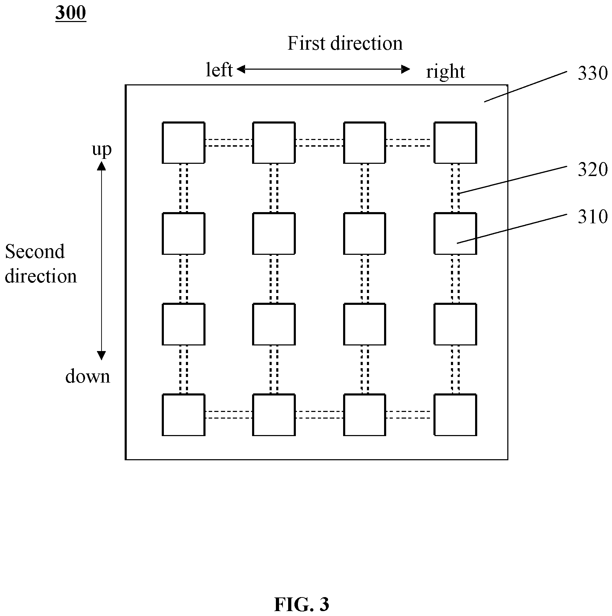

FIG. 3 is a schematic diagram illustrating an electrode structure according to some embodiments of the present disclosure.

In some embodiments, a count of connectors 320 disposed along a first direction in an electrode structure 300 is smaller than a count of the connectors 320 disposed along a second direction in the electrode structure 30, and a first elasticity coefficient of the electrode structure 300 along the first direction is greater than an elasticity coefficient of the electrode structure 300 along a second direction, so that a deformable amount of the electrode structure 300 along the first direction is greater than the deformable amount along the second direction. Electrodes 310, the connectors 320, and a substrate 330 of the electrode structure 300 are similar to the electrodes 110, the connectors 120, and the substrate 130 of the electrode structure 100, respectively. More descriptions regarding the electrodes 310, the connectors 320, and the substrate 330 of the electrode structure 300 can be found in the relevant descriptions regarding the electrodes 110, the connectors 120, and the substrate 130 of the electrode structure 100, which are not repeated herein.

In some embodiments, the adjacent electrodes 310 disposed along the second direction in each column are connected through the connector 320, and a portion of the adjacent electrodes disposed along the first direction are connected to each other through the connector 320, such that a count of the connectors 320 disposed along the second direction in the electrode structure 300 is greater than a count of the connectors 320 disposed along the first direction in the electrode structure 300, and a first elasticity coefficient of the electrode structure 300 along the first direction is smaller than a second elasticity coefficient of the electrode structure 300 along the second direction. In such cases, the deformable amount of the electrode structure 300 along the first direction is greater than the deformable amount along the second direction.
Specifically, when a count of rows disposed along the first direction and connected through the connectors 320 in the electrode structure 300 is less than a count of columns disposed along the second direction and connected through the connectors 320 in the electrode structure 300, the first elasticity coefficient of the electrode structure 300 along the first direction may be smaller than the second elasticity coefficient of the electrode structure 300 along the second direction, such that the deformable amount of the electrode structure 300 along the first direction is greater than the deformable amount along the second direction. For example, as shown in FIG. 3, the count of the connectors 320 300 disposed along the first direction in the electrode structure is 6, and the count of the connectors 320 disposed along the second direction in the electrode structure is 12. The 12 connectors 320 disposed along the second direction in the electrode structure are used to connect all two adjacent electrodes 310 disposed along the second direction in the electrode structure 300, 3 connectors 320 of the 6 connectors 320 disposed along the first direction in the electrode structure 300 are used to connect 4 electrodes 310 distributed in a first row from top to bottom in the electrode structure 300, and the other 3 connectors 320 are used to connect 4 electrodes 310 distributed in a last row from top to bottom in the electrode structure 300. In some embodiments, the count of connectors 320 disposed along the first direction in the electrode structure 300 is 3, and under the condition that all the electrodes 310 are capable of being electrically conductive to each other, the 3 connectors 320 are used to connect 4 electrodes 310 distributed in the same row in the electrode structure 300, or used to connect two adjacent electrodes 310 distributed in different rows in the electrode structure 300, wherein 4 electrodes in the electrodes 210 connected through the 3 connectors are distributed in different columns. For example, under the condition that all the electrodes 310 are capable of being electrically conductive to one another, the first connector 320 of the 3 connectors 320 is used to connect two adjacent electrodes 310 distributed in the first row, the second connector 320 of the 3 connectors 320 is used to connect two electrodes 310 distributed adjacent to each other in the second row, and the third connector 320 of the 3 connectors 220 is used to connect two electrodes 310 distributed adjacent to each other in the third row. 4 electrodes 210 of the electrodes 310 connected by the 3 connectors need to be distributed in different columns such that all the electrodes 310 are electrically conductive. In some embodiments, under the condition that all the electrodes 310 are capable of being electrically conductive to one another, the count of the connectors 320 disposed along the first direction in the electrode structure 300 is greater than 3 and less than the count of connectors 320 disposed along the first direction in the electrode structure 300. For example, the count of the connectors 320 disposed along the first direction in the electrode structure 300 may be 4, 5, 6, 7, etc. In some embodiments, under the condition that all the electrodes 310 are capable of being electrically conductive, only a portion of two adjacent electrodes 310 disposed along the second direction in the electrode structure 300 are connected through the connector 320, as long as the count of the connectors 320 disposed along the first direction is smaller than the count of the connectors 320 disposed along the second direction. It is understood that the electrodes 310 in each row of the electrode structure 300 refer to a plurality of electrodes 310 disposed along a direction parallel to the first direction and in the same straight line in the electrode structure 300. It is noted that the count of the electrodes 310 and the count of the connectors 320 shown in FIG. 3 is only for example and is not intended to be limiting, and when the count of the electrodes of the electrode structure is other counts (e.g., 100, 200, 500, 1000, etc.), the count of the connectors disposed along the first direction in the electrode structure and the count of the connectors disposed along the second direction in the electrode structure are designed with reference to the count of the connectors 320 disposed along the first direction in the electrode structure 300 and the count of the connectors 320 disposed along the second direction in the electrode structure 300.

In the embodiments of the present disclosure, each electrode (e.g., the electrode 110, 210, or 310) of the electrode structure (e.g., the electrode structure 100, 200, or 300) and one or more electrodes adjacent to the electrode are connected at least through the connector (e.g., the connector 120, 220, or 320) to realize the flexible connection with a great deformation range. When the electrodes are used for collecting physiological signals of human skin, the human body may have a better sense of comfort. In some embodiments, the connector includes a conductive structure, the conductive structure is electrically conductive, and each of the plurality of electrodes are connected to at least another of the plurality of electrodes through the conductive structure. In this way, electrical conduction is achieved between the two connected electrodes, and a displacement relative to each other is enabled between the two connected electrodes. In some embodiments, to enable the conductive structure and the electrodes to be better adapted to different size and shape requirements, a natural length of the conductive structure is greater than an initial distance between the connected electrodes. In some embodiments, to make the natural length of the conductive structure match a tensile deformation of the electrode structure, and since a stretching amount of the electrode structure is not too great, a ratio of the natural length of the conductive structure to the distance between the connected electrodes is in a range of 1.5-10.

In some embodiments, the conductive structure is an elastic conductive structure that is elastically stretchable along the axial direction. The conductive structure being elastically stretchable along the axial direction refers to that the conductive structure elongates or shortens in the axial direction thereof when subjected to an external force and restores to the initial length after the external force disappears. In some embodiments, the conductive structure has a deformable amount that directly affects the first elasticity coefficient of the electrode structure in the first direction and/or the second elasticity coefficient in the second direction. The deformable amount of the conductive structure refers to a percentage of a difference between a maximum length of the conductive structure after an elastic stretch in the axial direction when the conductive structure is about to undergo plastic deformation (or fracture) and the initial length of the conductive structure. In some embodiments, the greater the deformable amount of the conductive structure, the smaller the first elasticity coefficient of an electrode structure 500 along the first direction and/or the second elasticity coefficient of the electrode structure 500 along the second direction, and the greater the deformable amounts of the electrode structure 500 along the first direction and/or along the second direction. In some embodiments, the deformable amount of the conductive structure is in a range of 5%-200%. In some embodiments, the deformable amount of the conductive structure is in a range of 20%-200%. In some embodiments, the deformable amount of the conductible structure is in a range of 50%-200%. In some embodiments, the deformable amount of the conductible structure is in a range of 100%-200%.

In some embodiments, the conductive structure and the electrodes are separated structures, and two ends of the conductive structure are connected to two electrodes through bonding, welding, removable connections, etc. In some embodiments, the two ends of the conductive structure and the two electrodes are connected to together through a plug-in connection. In some embodiments, the two ends of the conductive structure are disposed with styli, the electrodes are disposed with sockets, and the styli are inserted into the sockets to achieve the plug-in connection between the two ends of the conductive structure and the electrodes, or, the two ends of the conductive structure are disposed with sockets and the styli are disposed at the electrodes. In some embodiments, the two ends of the conductive structure and the two electrodes are connected through a snap connection. In some embodiments, the two ends of the conductive structure are disposed with bumps, the electrodes are disposed with slots, and the bumps are embedded in the slots to realize the snap connection between the two ends of the conductive structure and the electrodes, or, the two ends of the conductive structure are disposed with slots and the electrodes are disposed with bumps. The removable connection between the conductive structure and the electrodes enables the electrode structure to be composed and disassembled at will to adapt to different size and shape requirements. When such an electrode structure is applied to a wearable device, by changing the size and a deformation of the electrode structure, the wearable device is suitable for adults or children. In some embodiments, the plurality of electrodes are divided into two groups. One group of the electrodes (which is referred to as a first electrode group) is fixedly disposed on a wearing portion of the wearable device, and the other group of the electrodes (which is referred to as a second electrode group) is detachably connected to the wearing portion of the wearable device. In some embodiments, the electrodes in the second electrode group have electrode plugs (e.g., the aforementioned styli, bumps, and other structures), and correspondingly, the wearable device is disposed with electrode interfaces (e.g., the aforementioned sockets, slots, and other structures). The electrode plugs and the electrode interfaces are used to realize the connection and the disconnection between the electrodes and the wearable device by plugging and unplugging. In such cases, the second electrode group is arbitrarily connected with the wearable devices of different sizes to realize more usage possibilities, and as the second electrode group can be reused, the replacement of the wearable device is also realized at a lower cost. In some embodiments, the second electrode group includes at least two electrodes connected to each other through the conductive structure. In some embodiments, the plurality of electrodes may not include the first electrode group such that all the electrodes on the wearable device are removable electrodes.

The conductive structure is a key to improve the elasticity coefficient of the electrode structure, and the conductive structure in the embodiments of the present disclosure are described in detail below in conjunction with the accompanying drawings.

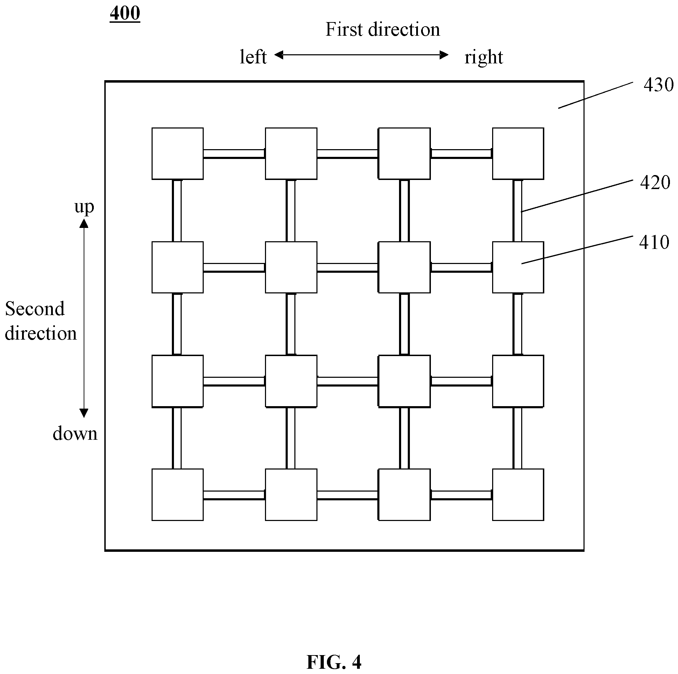

FIG. 4 is a schematic diagram illustrating an electrode structure according to some embodiments of the present disclosure.

In some embodiments, a conductive structure of the embodiments of the present disclosure includes one or more connecting electrodes 420 as shown in FIG. 4. In some embodiments, each of the plurality of electrodes 410 of the electrode structure 400 is flexibly connected to at least one of the plurality of electrodes 410 through the one or more connecting electrodes 420, as shown in FIG. 4, and the one or more connecting electrodes 420 electrically conduct the connected electrodes 410. In some embodiments, each of the plurality of electrodes 410 is flexibly connected to all of the electrodes 410 adjacent thereto through the one or more connecting electrodes 420, as shown in FIG. 4. In some embodiments, under the condition that all the electrodes 410 are capable of being electrically conductive to each other, each electrode 410 is flexibly connected to at least one electrode 410 adjacent thereto through the one or more connecting electrodes 420. The plurality of electrodes 410 and the substrate 430 of the electrode structure 400 are similar to the electrodes 110 and the substrate 130 of the electrode structure 100, respectively, and more descriptions regarding the electrodes 410, the substrate 430 of the electrode structure 400 can be found in the relevant descriptions regarding the electrode 110 and the substrate 130 in the electrode structure 100, which is not repeated herein. In some embodiments, the connecting electrode 420 refers to a connector that acts as a conductive medium as the electrode 410 and is connected between two adjacent electrodes 410. In some embodiments, materials of the one or more connecting electrodes 420 and the electrodes 410 are the same or different. In some embodiments, the one or more connecting electrodes 420 are configured to electrically conduct the adjacent electrodes 410, or perform a same function as the electrodes 410, i.e., to collect EMG signals when applied to a skin.

In some embodiments, by controlling a maximum size of the one or more connecting electrodes 420 in a first direction and/or a maximum size of the one or more connecting electrodes 420 in a second direction, the one or more connecting electrodes 420 are capable of achieving flexible connections between the connected electrodes 410. In some embodiments, the maximum size of the one or more connecting electrodes 420 disposed along the first direction in the first direction is greater than the maximum size of the one or more connecting electrodes 420 disposed along the first direction in the second direction in the electrode structure 400, and the greater a ratio of the maximum size of the one or more connecting electrodes 420 disposed along the first direction in the first direction to the maximum size of the one or more connecting electrodes 420 disposed along the first direction in the second direction, the smaller the elasticity coefficient of the one or more connecting electrodes 420 disposed along the first direction, the smaller the first elasticity coefficient of the electrode structure 400 along the first direction, and the greater the deformable amount of the electrode structure 400 along the first direction. In some embodiments, the maximum size of the one or more connecting electrodes 420 of the electrode structure 400 disposed along the second direction in the first direction is smaller than the maximum size of the one or more connecting electrodes 420 disposed along the second direction in the second direction, and the smaller a ratio of the maximum size of the one or more connecting electrodes 420 disposed along the second direction in the first direction to the maximum size of the one or more connecting electrodes 420 disposed along the second direction in the second direction, the smaller the elasticity coefficient of the one or more connecting electrodes 420 disposed along the second direction, the smaller the second elasticity coefficient of the electrode structure 400 along the second direction, and the greater the deformable amount of the electrode structure 400 along the second direction. In some embodiments, the maximum size of the one or more connecting electrodes 420 disposed along the first direction in the second direction in the electrode structure 400 is smaller than the maximum size of the electrodes 410 disposed along the first direction in the second direction. In some embodiments, the maximum size of the one or more connecting electrodes 420 disposed along the second direction in the first direction is smaller than the maximum size of the electrodes 410 disposed along the second direction in the first direction.

In some embodiments, the one or more connecting electrodes 420 and the electrodes 410 are separated structures, and the two ends of each of the one or more connecting electrodes 420 are connected to the two adjacent electrodes 410 through bonding, welding, etc. In some embodiments, the two ends of each of the one or more connecting electrodes 420 are detachably connected to two electrodes. In some embodiments, the two ends of each of the one or more connecting electrodes 420 are connected to the two electrodes 410 through a plug-in connection. In some embodiments, the two ends of the one or more connecting electrodes 420 are connected to the two electrodes 410 through a snap connection. Specific descriptions for the plug-in connection and snap connection may be found in descriptions regarding the conductive structures. In some embodiments, to enable the one or more connecting electrodes 420 and the electrodes 410 to better adapt to different size and shape requirements, a natural length of the connecting electrode 420 is greater than an initial distance between the connected electrodes. In some embodiments, to enable the one or more connecting electrodes 420 and the electrodes 410 better adapt to different size and shape requirements, the one or more connecting electrodes 420 are flexible electrodes. In some embodiments, the deformable amount the connecting electrode 420 in an axial direction is in a range of 5%-200%.

In some embodiments, the connecting electrode 420 and one or both of the electrodes 410 connected to the connecting electrode 420 are of an integrated structure, or the connecting electrode 420 is regarded as a portion of the electrode 410. In some embodiments, the electrode structure 400 is integrated, i.e., all of the electrodes 410 in the electrode structure 400 and the one or more connecting electrodes 420 are in an integrated structure, the electrodes 410 and the one or more connecting electrodes 420 are connected without other connections. For example, an entire electrode sheet is skeletonized (e.g., laser cut, stamped, etc.) to obtain a plurality of electrodes 410 and connecting electrodes 420 in an integrated structure, i.e., the electrode structure 400. In some embodiments, the skeletonized electrode sheet forms a grid structure, and the electrode sheet has a plurality of regularly or irregularly arranged meshes. A thinner portion (a portion disposed along the first direction with a small size in the second direction, or a portion disposed along the second direction with a small size in the first direction) of the electrode sheet is the connecting electrode 420 and a thicker portion (a portion disposed along the first direction with a great size in the second direction, or a portion disposed along the second direction with a great size in the first direction) of the electrode sheet is the electrode 410. It is noted that the connecting electrode 420 applies not only to the electrode structure 400 shown in FIG. 4, but also to the connector 120 in the electrode structure 100, the connector 220 in the electrode structure 200, and the connector 320 in the electrode structure 300.

FIG. 5 is a schematic diagram illustrating an electrode structure according to some embodiments of the present disclosure.

In some embodiments, a conductive structure in the embodiments of the present disclosure includes one or more wires 520 as shown in FIG. 5. In some embodiments, each of a plurality of electrodes 510 in an electrode structure 500 is flexibly connected to at least one of the plurality of electrodes 510 through the one or more wires 520, and the one or more wires 520 electrically conducts the connected electrodes 510, as shown in FIG. 5. In some embodiments, each of the plurality of electrodes 510 is flexibly connected to all of the electrodes 510 adjacent thereto through the one or more wires 520, as shown in FIG. 5. In some embodiments, under the condition that all the electrodes 510 are capable of being electrically conductive to each other, each of the plurality of electrodes 510 is flexibly connected to at least one electrode 510 adjacent thereto through the one or more wires 520. The electrodes 510 and the substrate 530 of the electrode structure 500 are similar to the electrodes 110 and the substrate 130 of the electrode structure 100, and more descriptions regarding the electrodes 510 and the substrate 530 may be found in the relevant descriptions regarding the electrodes 110 and the substrate 130 of the electrode structure 100, which are not repeated herein. In some embodiments, the wire 520 refers to a wire-like structure with a certain electrical conductivity.

In some embodiments, to ensure that the one or more wires 520 are capable of realizing the flexible connection between the connected electrodes 510, the one or more wires 520 are elastically stretchable along axial directions thereof. The wire 520 being elastically stretchable along the axial directions thereof refers to that the wire 520 is elongated or shortened in the axial direction thereof when subjected to an external force and restores to the initial length after the external force disappears. In some embodiments, a material of the one or more wires 520 includes at least one of fibers coated with a conductive metal, fibers deposited with conductive substances, and polymer composites of mixed conductive material. Exemplary conductive metals include gold, silver, iron, and copper, etc. Exemplary conductive materials include electrically conductive metals, carbon, and graphene, etc. These materials ensure that the one or more wires 520 can electrically conduct the connected electrodes 510, and allow the one or more wires 520 to be elastically stretchable along axial directions thereof.

In some embodiments, the wire 520 and the electrodes 510 are separated structures, and two ends of the wire 520 are connected to two adjacent electrodes 510 through bonding, welding, etc. In some embodiments, the two ends of the wire 520 are detachably connected to the two electrodes 510.

In some embodiments, the deformable amounts of the one or more wires 520 directly affect a first elasticity coefficient of the electrode structure 500 along a first direction and/or a second elasticity coefficient along a second direction. In some embodiments, the deformable amounts of the one or more wires 520 refer to a percentage of a difference between a maximum length of the wire 520 after elastic elongation in the axial direction when the wire 520 is about to undergo plastic deformation (e.g., fracture) and the initial length of the wire 520. In some embodiments, the greater the deformable amounts of the one or more wires 520, the smaller the first elasticity coefficient of the electrode structure 500 along the first direction and/or the second elasticity coefficient of the electrode structure 500 along the second direction, and the greater the deformable amounts of the electrode structure 500 along the first direction and/or the second direction. In some embodiments, the deformable amount of the one or more wires 520 is 5%-200%. In some embodiments, the deformable amount of the one or more wires 520 is in a range of 20%-200%. In some embodiments, the deformable amount of the one or more wires 520 is in a range of 50%-200%. In some embodiments, the deformable amount of the one or more wires 520 is in a range of 100%-200%. It is noted that the one or more wires 520 apply not only to the electrode structure 500 shown in FIG. 5, i.e., the connector 120 in the electrode structure 100, but also to the connector 220 of the electrode structure 200, and the connector 320 of the electrode structure 300. In some alternative embodiments, the one or more wires 520 are liquid wires formed by a liquid conductor. Adjacent electrodes 510 are connected to each other through the liquid conductor, which provides a better flexible connection effect.

FIG. 6 is a schematic diagram illustrating an electrode structure according to some embodiments of the present disclosure.

In some embodiments, the connector (e.g., the connectors 120, 220, or 320) in the embodiments of the present disclosure includes one or more wires 620 shown in FIG. 6. In some embodiments, as shown in FIG. 6, each of a plurality of electrodes 610 in the electrode structure 600 is flexibly connected to at least one of the plurality of electrodes 610 through the one or more wires 620, and the one or more wires 620 electrically conduct the connected electrodes 610. In some embodiments, each of the plurality of electrodes 610 is flexibly connected to all the electrodes 610 adjacent thereto through the one or more wires 620, as shown in FIG. 6. In some embodiments, under the condition that all of the electrodes 610 are capable of being electrically conductive to each other, each of the plurality of electrodes 610 is flexibly connected to at least one of the electrodes 610 adjacent thereto through the one or more wires 620. The electrodes 610 and the substrate 630 of the electrode structure 600 are similar to the electrodes 110 and the substrate 130 of the electrode structure 100, and more descriptions regarding the electrodes 610 and the substrate 630 in the electrode structure 600 can be found in the relevant description regarding the electrode 110 and the substrate 130 of the electrode structure 100, which are not repeated herein.

In some embodiments, to ensure that the one or more wires 620 are capable of realizing a flexible connection between the connected electrodes 610, a natural length of the wire 620 is greater than an initial distance between the connected electrodes 610. The natural length of the wire 620 refers to a length of the wire 620 when the wire 620 is unfolded into a straight line, and the initial distance between the connected electrodes 610 refers to a distance between two adjacent electrodes 610 to which the wire 620 is connected when the electrode structure 600 is not deformed (e.g., telescoped or flexed along a first direction or a second direction, etc.) by an external force. In some embodiments, the natural length of the wire 620 is greater than the distance between the connected electrodes 610 such that when the wire 620 is connected between two adjacent electrodes 610, the wire 620 includes at least one bending portion. When the substrate 630 is stretched by a force along the first direction or the second direction, the bending portion of the wire 620 may be unfolded, thereby reducing obstruction to the substrate 630 when the substrate 630 is stretched and deformed, and achieving the flexible connection between the connected electrodes 610. In some embodiments, a ratio of the natural length of the wire 620 to the initial distance between the connected electrodes 610 is in a range of 1.5-10. In some embodiments, the ratio of the natural length of the wire 620 to the initial distance between the connected electrode 610 is 2-10. In some embodiments, the ratio of the natural length of the wire 620 to the initial distance between the connected electrode 610 is in a range of 5-10. In some embodiments, the wire 620 is made from a rigid material with a certain conductive capacity, for example, the material of the wire 620 is a metallic material such as gold, silver, iron, copper, etc. In some embodiments, the wire 620 is made of the same material as the wire 520. It is noted that the wire 620 applies not only to the electrode structure 600 shown in FIG. 6, i.e., the connector 120 in the electrode mechanism 100, but also to the connector 220 of the electrode structure 200, and the connector 320 in the electrode structure 300.

Embodiments of the present disclosure also provide a wearable device, which is configured to be worn on a human body to collect physiological signals of the human body, and ensure that the human body has a good sense of comfort. The wearable device provided by the embodiments of the present disclosure is described in detail below in conjunction with the accompanying drawings.

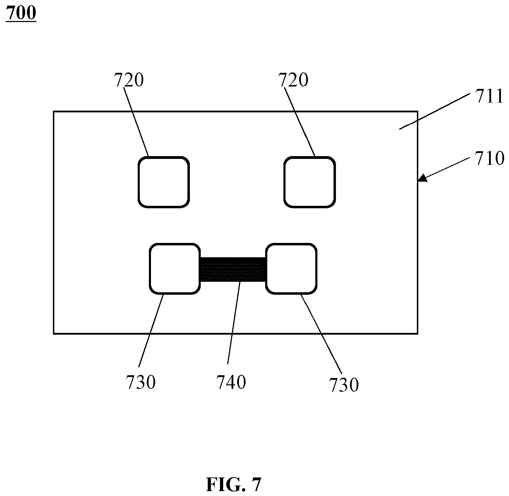

FIG. 7 is a schematic diagram illustrating a wearable device according to some embodiments of the present disclosure.

As shown in FIG. 7, a wearable device 700 includes a wearing portion 710, at least two first electrodes 720, and at least two second electrodes 730. The wearing portion 710 includes a substrate 711, the at least two first electrodes 720 are spaced apart on the substrate 711 and are configured to fit to the skin to collecting physiological signals (e.g., EMG signals, ECG signals, etc.), and the at least two second electrodes 730 are spaced apart on the substrate 711 and electrically connected to each other through a connector 740 to provide a reference voltage for the collected physiological signals.

The wearing portion 710 is configured to be worn on a user's body. In some embodiments, the wearing portion 710 is a top (e.g., a t-shirt, a vest, a tank top, a jacket, etc.) that is worn on the upper body of a user. In some embodiments, the wearing portion 710 is pants (e.g., trousers, shorts, etc.) worn on the lower body of the user. In some embodiments, the wearing portion 710 is a leg loop or a belt worn on the user's leg or waist, respectively. In some embodiments, the wearing portion 710 is a bracelet, a helmet, etc. worn on the user's hand or head, respectively. In some embodiments, the substrate 711 refers to a surface of the wearing portion 710 that is fitted to the user's body. In some embodiments, the wearing portion 710 has a consistent or approximately consistent deformable amount with the first electrode 720 and/or the second electrode 730, which ensures that a portion of the wearing portion 710 where the first electrode 720 and/or the second electrode 730 are disposed is less likely to be damaged.

In some embodiments, when the user wears the wearing portion 710, the at least two first electrodes 720 are fitted to both sides of a median sagittal plane of the user, e.g., the at least two first electrodes 720 are fitted to the user's chest, waist, etc. on both sides of the median sagittal plane, or the at least two first electrodes 720 are fitted to the user's left and right hands, left and right legs, etc., respectively. In some embodiments, the at least two first electrodes 720 are symmetrical with respect to the median sagittal plane of the user, which facilitates an elimination or suppression of an interference of noise (e.g., motion artifacts) in the collected physiological signals (e.g., the ECG signals), and improves a quality of the physiological signals. In some embodiments, the at least two first electrodes 720 are not symmetrical with respect to the median sagittal plane of the user. In some embodiments, the median sagittal plane of the user refers to a plane that passes through a median line of the user's body. The median line of the user's body is determined based on a line from the nose tip of the user to the middle of two nipples, and a line from the middle of the two nipples to the middle of an abdominal umbilicus or a line from the middle of the abdominal umbilicus to a middle of the symphysis pubis joint.

In some embodiments, when the at least two first electrodes 720 are used to collect the EMG signals, the at least two first electrodes 720 are fitted to the skin along a direction of muscle fibers of the user. In some embodiments, the first electrodes 720 may be one piece of electrode, e.g., the first electrodes 720 are similar to the electrodes (e.g., the electrodes 110, 210, 310, 410, 510, or 610) in the embodiments of the present disclosure. In some embodiments, the first electrodes 720 may be an electrode structure provided in the embodiments of the present disclosure (e.g., the electrode structure 100, 200, 300, 400, 500, or 600), and the electrode structure provided in the embodiments of the present disclosure has a great deformable amount. When the electrode structure is disposed on the wearing portion 710 to fit with the user's skin, the user's movements are not restricted and force generation is not impeded, so as to provide the user with a better sense of comfort and make it easy for the user to put on or take off the wearable device, which brings the user with a better sense of comfort.

In some embodiments, the at least two second electrodes 730 serve as reference electrodes to provide a reference voltage for the physiological signals collected by the at least two first electrodes 720, thereby facilitating the elimination or suppression of the interference of noise (e.g., the motion artifacts, an operation frequency, etc.) in the physiological signals, and improving the quality of the physiological signals. For example, the voltage generated by the second electrodes 730 is used as a reference voltage for an amplifier during an amplification of the signals collected by the first electrodes 720.

In some embodiments, the at least two second electrodes 730 are symmetrical with respect to the median sagittal plane of the user when the user is wearing the wearing portion 710, which improves the consistency of the deformable amounts (or referred to as elasticity) of the wearing portion 710 on both sides of the median sagittal plane of the user, thereby facilitating the elimination or suppression of the interference of noise (e.g., the motion artifacts) in the physiological signals collected by the at least two first electrodes 720, and improving the quality of the physiological signals. In some embodiments, the second electrodes 730 may be one piece of electrode, e.g., the second electrodes 730 may be similar to the electrodes (e.g., the electrodes 110, 210, 310, 410, 510, or 610) in some embodiments of the present disclosure. The second electrodes 730 may be an electrode structure provided in the embodiments of the present disclosure (e.g., the electrode structure 100, 200, 300, 400, 500, or 600).

In some embodiments, the connector 740 not only realizes an electrical connection between the at least two second electrodes 730 to maintain a uniform low reference voltage, but also realizes a flexible connection between the at least two second electrodes 730, which enables the overall structure formed by the at least two second electrodes 730 and the connector 740 connected thereto to have a relatively great deformable amount. When the overall structure is disposed on the wearing portion 710 to fit with the user's skin, the user's movements are not restricted, and the force generation is not impeded, so that the user has a better sense of comfort and it is easy for the user to put on or take off the wearable device. In some embodiments, the connector 740 is similar to the connector (e.g., the connector 120, 220, or 320) in the embodiments of the present disclosure, and the connector 740 may be the connecting electrode 420 shown in FIG. 4, the wire 520 shown in FIG. 5, or the wire 620 shown in FIG. 6. More detailed descriptions regarding the connector 740 can be found in relevant descriptions regarding the connecting electrode 420 shown in FIG. 4, the wire 520 shown in FIG. 5, or the wire 620 shown in FIG. 6, which are not repeated herein.

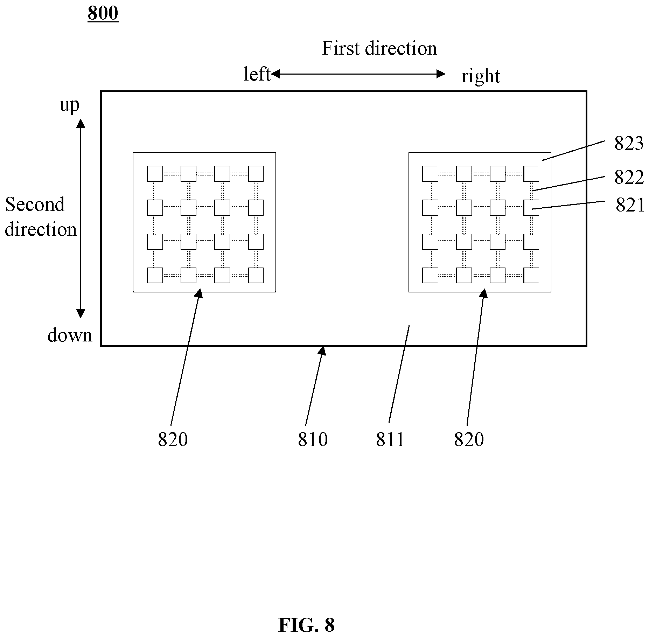

FIG. 8 is a schematic diagram illustrating a wearable device according to some embodiments of the present disclosure.

As shown in FIG. 8, a wearable device 800 includes a wearing portion 810 and at least two electrode structures 820. The wearing portion 810 includes a substrate 811 that fits a user's body, and the at least two electrode structures 820 are spaced apart on the substrate 811 for fitting to a skin to collect physiological signals. The wearing portion 810 and the substrate 811 are similar to the wearing portion 710 and the substrate 711, respectively of the wearable device 700. More descriptions regarding the wearing portion 810 and the substrate 811 can be found in the description regarding the wearing portion 710 and the substrate 711 in the wearable device 700, which are not repeated herein.

The electrode structure 820 includes a plurality of electrodes 821, each of the plurality of electrodes 821 is electrically connected to at least another of the plurality of electrodes 821 through connectors 822, and the electrode structure 820 is used to collect electrical signals from the user's body (e.g., electrical signals from a same target muscle). The electrode 821 is similar to the electrode 110 shown in FIG. 1, and more descriptions regarding the electrode 821 may be found in the relevant descriptions regarding the electrode 110, which are not repeated herein. In some embodiments, the physiological signals are determined based on the electrical signals collected by the at least two electrode structures 820 from the corresponding target muscle. For example, the at least two electrode structures 820 collect different electrical signals at different positions (e.g., different positions along a direction of the muscle fibers) of the same target muscle in the user's body. The electrical signals are potentials corresponding to the target muscle. A difference in the potentials between different positions of the target muscle can reflect a force generation of the target muscle. In some embodiments, the at least two electrode structures 820 are symmetrically or asymmetrically fitted on either side of the user's body with respect to a median sagittal plane of the user. In some embodiments, the electrode structures 820 have consistent or approximately consistent deformable amounts with the wearing portion 810, which ensures that the portion of the wearing portion 810 on which the electrode structures 820 are disposed is less likely to be damaged.

In some embodiments, the electrode structures 820 are similar to the electrode structures provided in the embodiments of the present disclosure (e.g., the electrode structures 100, 200, 300, 400, 500, or 600), e.g., the electrode structures 820 are similar to the electrode structure 100, as shown in FIG. 8. More descriptions regarding the electrode structures 820 and the electrodes 821 may be found in the relevant descriptions regarding the electrode structure 100 shown in FIG. 1, the electrode structure 200 shown in FIG. 2, the electrode structure 300 shown in FIG. 3, the electrode structure 400 shown in FIG. 4, the electrode structure 500 shown in FIG. 5, or the electrode structure 600 shown in FIG. 6, which are not repeated herein. The connector 822 is similar to the connector (e.g., the connector 120, 220, or 320) in the embodiments of the present disclosure, and the connector 822 may be the connecting electrode 420 shown in FIG. 4, the wire 520 shown in FIG. 5, or the wire 620 shown in FIG. 6 and configured to achieve a flexible connection between the electrodes 821 to which the connector 822 is connected, so as to make the electrode structure 820 has a great deformable amount (or referred to as an elasticity). When the connector 822 is disposed on the wearing portion 810 to fit the user's skin, the user's movement is not restricted and the force generation is not impeded, so as to provide the user with a better sense of comfort and make it easy for the user to put on or take off the wearable device. More descriptions regarding the connector 822 can be found in the relevant description regarding the connecting electrode 420 shown in FIG. 4, the wire 520 shown in FIG. 5, or the wire 620 shown in FIG. 6, respectively, which are not repeated herein.

In some embodiments, the electrode structure 820 further includes a substrate 823, and the plurality of electrodes 821 are spaced apart on the substrate 823 along a first direction and a second direction, respectively. In some embodiments, the substrate 823 is a portion of the substrate 811. In some embodiments, the substrate 823 is disposed separately with respect to the substrate 811, e.g., a side of the substrate 823 away from the electrodes 821 is connected to the substrate 811, so that the electrode structure 820 is attached to the substrate 811. More descriptions regarding the substrate 823 may be found in the descriptions regarding the substrate 130 shown in FIG. 1, which is not repeated herein.

The basic concepts have been described above, and it is apparent to those skilled in the art that the foregoing detailed disclosure is intended as an example only and does not constitute a limitation of the present disclosure. Although not explicitly stated here, those skilled in the art may make various modifications, improvements and amendments to the present disclosure. These alterations, improvements, and modifications are intended to be suggested by the present disclosure, and are within the spirit and scope of the exemplary embodiments of the present disclosure.

Moreover, certain terminology has been used to describe embodiments of the present disclosure. As in "an embodiment," "one embodiment," and/or " some embodiments" means a feature, structure, or characteristic associated with at least one embodiment of the present disclosure. Accordingly, it should be emphasized and noted that two or more references to "an embodiment" or "one embodiment" in different positions in the present disclosure are not necessarily refer to the same embodiment. In addition, some features, structures, or features in one or more embodiments of the present disclosure may be appropriately combined.

Similarly, it should be appreciated that in the foregoing description of embodiments of the present disclosure, various features are sometimes grouped together in a single embodiment, figure, or description thereof for the purpose of streamlining the disclosure and aiding in the understanding of one or more of the various embodiments. However, the present disclosure does not mean that the present disclosure object requires more features than the features mentioned in the claims. Rather, claimed subject matter may lie in less than all features of a single foregoing disclosed embodiment.

At last, it should be understood that the embodiments described in the present disclosure are merely illustrative of the principles of the embodiments of the present disclosure. Other modifications that may be employed are within the scope of the present disclosure. Thus, by way of example, but not of limitation, alternative configurations of the embodiments of the present disclosure may be utilized in accordance with the teachings herein. Accordingly, the embodiments of the present disclosure are not limited to that precisely as shown and described.

## Claims

1. An electrode structure, comprising: a plurality of electrodes, wherein
each of the plurality of electrodes is flexibly connected to at least another of the plurality of electrodes through a connector,
the connector allows electrical conduction between the connected electrodes.

2. The electrode structure of claim 1, wherein the connector includes a conductive structure, and the each of the plurality of electrodes is flexibly connected to the at least another of the plurality of electrodes through the conductive structure.

3. The electrode structure of claim 2, wherein the conductive structure has a deformable amount of 5% to 200% along an axial direction of the conductive structure.

4. The electrode structure of claim 2, wherein a natural length of the conductive structure is greater than an initial distance between the connected electrodes.

5. The electrode structure of claim 4, wherein a ratio of the natural length of the conductive structure to the initial distance between the connected electrodes is 1.5 to 10.

6. The electrode structure of any one of claims 2 to 5, wherein the conductive structure is detachably connected to the connected electrodes.

7. The electrode structure of any one of claims 2 to 6, wherein the conductive structure is connected to each of the connected electrodes through a plug-in connection.

8. The electrode structure of any one of claims 2 to 7, wherein
the conductive structure includes one or more wires,
the each of the plurality of electrodes is flexibly connected to the at least another of the plurality of electrodes through at least one of the one or more wires, and
the at least one wire allows electrical conduction between the connected electrodes.

9. The electrode structure of claim 8, wherein a material of the one or more wires includes at least one of fibers coated with conductive metal, fibers deposited with conductive substances, and polymer composites of mixed conductive material.

10. The electrode structure of any one of claims 2 to 7, wherein
the conductive structure includes one or more connecting electrodes,
the each of the plurality of electrodes is flexibly connected to the at least another of the plurality of electrodes through at least one of the one or more connecting electrodes, and
the at least one connecting electrode allows electrical conduction between the connected electrodes.

11. The electrode structure of claim 10, wherein the one or more connecting electrodes and the plurality of electrodes are an integrated structure.

12. The electrode structure of any one of claims 2 to 11, wherein for each of the plurality of electrodes,
a ratio of a maximum size of the electrode along a first direction to a maximum size of the electrode in a second direction is 0.1 to 10, wherein the first direction is perpendicular to the second direction.

13. The electrode structure of any one of claims 2 to 11, wherein
the electrode structure further includes a substrate,
the plurality of electrodes being arranged on the substrate along a first direction and a second direction, respectively, and
the electrode structure has different elasticity coefficients along the first and the second direction respectively.

14. The electrode structure of claim 1, wherein each of the plurality of electrodes has an area of 1 mm² to 20 mm².

15. The electrode structure of claim 1, wherein a ratio of the area of the electrode structure to a sum of the areas of the plurality of electrodes is 1 to 1000.

16. A wearable device, comprising:
a wearable wearing portion, including a substrate fitting a user's body;
at least two first electrodes spaced apart on the substrate and configured to fit a skin to collect physiological signals, respectively; and
at least two second electrodes spaced apart on the substrate and electrically connected to each other through a connector, the at least two second electrodes being configured to fit the skin to provide a reference voltage for the physiological signals.

17. The wearable device of claim 16, wherein the at least two second electrodes are symmetrical with respect to a mid-sagittal plane of the user when the user wears the wearing portion.

18. A wearable device, comprising:
a wearable wearing portion, including a substrate fitting a user's body;
at least two electrode structures spaced apart on the substrate and configured to fit a skin to collect physiological signals, wherein
each of the at least two electrode structures includes a plurality of electrodes, each of the plurality of electrodes is electrically connected to at least another of the plurality of electrodes through a connector, and the electrode structure is configured to collect electrical signals from a same target muscle of the user's body.

19. The wearable device of claim 18, wherein at least a portion of the plurality of electrodes are detachably connected to the wearing portion.
